# EUROPEAN PATENT SPECIFICATION

(11) **EP 2 347 720 B1**
(45) Date of publication and mention of the grant of the patent: **17.05.2017**
(21) Application number: 11151576.3
(22) Date of filing: 20.01.2011
(51) Int. Cl.: A61B 17/04, A61B 17/06

(54) **Suture passer assembly**
Nahtdurchgangsanordnung
Ensemble formant passe-suture

(30) Priority: 20.01.2010 US 296718 P
(43) Date of publication of application: 27.07.2011
(73) Proprietor: Howmedica Osteonics Corp., Mahwah, NJ 07430 (US)
(72) Inventor: Wymann, Jeffrey, Naples FL 34108 (US); Yearsley, Ryan E., Eagle ID 83616 (US); Borchert, Jeremy Dirk, AUSTIN,TX 78717 (US); Sinnott, Margaret Mary, Logan UT 84321 (US)
(74) Representative: Regimbeau

(56) References cited:
- EP-A1- 1 498 075
- WO-A1-96/39948
- WO-A1-97/10756
- DE-A1- 4 235 602
- US-A1- 2002 103 493
- US-A1- 2007 060 953
- US-A1- 2007 225 735
- US-A1- 2007 270 885
- US-A1- 2008 208 221
- US-A1- 2009 177 039

## Description

### BACKGROUND OF THE INVENTION

The present invention relates to suture passer assemblies and methods of using same. More particularly, the present invention relates to a front-loading suture passer device having a distal end that is rotatable with respect to the handle.

There are known surgical instruments that allow a surgeon to pass a suture through tissue, for example during an arthroscopic rotator cuff repair. Such instruments may include a tissue grasping portion such as a pair of jaws that allows for the tissue to be grasped while a piercing instrument punctures through the tissue.

Published document US 2007/270885 discloses a suturing assembly and method, which is adapted to engage a suture by a bendable needle.

Published document EP 1498075 discloses a suture passer instrument which enables an easier positioning of the jaws carrying the suture.

Published document US2007/225735 discloses a system and method for passing a suture, comprising a pushing member which advances through a shaft in order to place a suture.

Published document US 2002/103493 discloses a surgical suture passer comprising a pair of jaws that enable the surgeon to manipulate a suture.

Published document DE 4235602 discloses a surgical instrument to engage a suture with a needle.

Published document WO96/39948 discloses a suture passing forceps that enables to insert a suture through a needle.

Published document WO 97/10756 discloses a surgical instrument which enables both tissue clamping and suturing. Published document US2007/060953 discloses a compact suture punch with malleable needle according to the preamble of claim 1.

The procedures in which these instruments are used are often arthroscopic in nature, and are often performed through a cannula, which may only be placed in certain positions and orientations due to the patient's anatomy. This requires that the surgical instrument be positioned according to the constraints of the surgical site, which limits the number of positions from which the surgical instrument may be operated. As a result, there are often constraints on the surgeon that result from the particulars of the surgery and the surgical instrument itself.

Therefore, a need exists for a surgical instrument and associated method of use that allows the surgeon greater flexibility in determining the operating position of the instrument. Such a device would allow the surgeon to perform a suturing procedure by operating a handle portion of the instrument from a multitude of positions with respect to the grasping portion of the instrument. Such a device would be more ergonomic and would allow a surgeon to exert greater control over the procedure. Such a device would also allow for greater flexibility in positioning a patient for surgery.

### BRIEF SUMMARY OF THE INVENTION

The invention is as disclosed in the appended set of claim. A first aspect of the present disclosure is a surgical instrument including a jaw portion extending along an axis and having a distal end and a proximal end, the distal end having an upper jaw and a lower jaw having a forked end, and a handle portion rotatably connected with the proximal end of the jaw portion such that the handle portion is configured to at least partially rotate about the axis with respect to the jaw portion.

In accordance with certain aspects of this first aspect, the instrument may further include a shaft portion extending along the axis between the handle portion and the jaw portion, the shaft portion being rotationally coupled to the proximal end of the jaw portion. The instrument may further include a needle assembly configured to pass through the shaft portion and the lower jaw, wherein the needle assembly is adapted to be passed along the axis by cooperation of the needle assembly with the handle portion. The needle assembly may be rotatable with respect to the handle portion. The lower jaw may include a non-circular needle channel, and the needle assembly may include a non-circular needle blade slidable within the needle channel, wherein the needle assembly is rotationally coupled with the jaw portion and rotationally connected to the handle portion due to the mating configuration between the needle channel and needle blade. The lower jaw may include a needle channel and the forked end may define first and second arms having a space therebetween that intersects the needle channel, wherein the space is configured to accept a suture for engagement with the needle assembly.

The shaft portion may include at least one spring-assisted bearing and the handle portion may include at least one depression configured to engage the at least one spring-assisted bearing in an orientation that temporarily locks the shaft portion with respect to handle portion. The at least one spring-assisted bearing and the at least one depression may be engageable in a finite number of orientations about the axis that temporarily lock the shaft portion with respect to handle portion. The handle portion may be further rotated with respect to the jaw portion through application of a force to overcome the connection between the at least one spring-assisted bearing and the at least one depression in the temporarily locked orientation. The handle portion may be configured to rotate at least 90 degrees about the axis with respect to the jaw portion. The handle portion may be configured to rotate at least 180 degrees about the axis with respect to the jaw portion. The handle portion may be configured to fully rotate about the axis with respect to the jaw portion.

A second aspect of the present disclosure is a suture passer assembly including a jaw portion extending along an axis and having a proximal end, a handle portion rotatably connected with the proximal end of the jaw portion such that the handle portion is configured to at least partially rotate about the axis with respect to the jaw portion, and a needle assembly configured to pass through the jaw portion and adapted to be passed along the axis by cooperation of the needle assembly with the handle portion.

In accordance with certain aspect of this second aspect, the instrument may further include a shaft portion extending along the axis between the handle portion and the jaw portion, the shaft portion being rotationally coupled to the proximal end of the jaw portion. The shaft portion may include at least one spring-assisted bearing and the handle portion may include at least one depression configured to engage the at least one spring-assisted bearing in an orientation that temporarily locks the shaft portion with respect to handle portion. The at least one spring-assisted bearing and the at least one depression may be engageable in a finite number of orientations about the axis that temporarily lock the shaft portion with respect to handle portion. The handle portion may be further rotated with respect to the jaw portion through application of a force to overcome the connection between the at least one spring-assisted bearing and the at least one depression in the temporarily locked orientation.

The handle portion may be configured to rotate at least 90 degrees about the axis with respect to the jaw portion. The handle portion may be configured to rotate at least 180 degrees about the axis with respect to the jaw portion. The handle portion may be configured to fully rotate about the axis with respect to the jaw portion. The needle assembly may be rotatable with respect to the handle portion. The jaw portion may include a lower jaw having a non-circular needle channel, and the needle assembly may include a non-circular needle blade slidable within the needle channel, wherein the needle assembly is rotationally coupled with the jaw portion and rotationally connected to the handle portion due to the mating configuration between the needle channel and needle blade. The jaw portion may include a lower jaw having a forked end and a needle channel, the forked end defining first and second arms having a space therebetween that intersects the needle channel, wherein the space is configured to accept a suture for engagement with the needle assembly.

A third aspect of the present disclosure is a method of using a surgical instrument including the steps of rotating a handle portion of the instrument with respect to a jaw portion of the instrument about an axis, the jaw portion extending along the axis and having a distal end and a proximal end, the distal end having an upper jaw and a lower jaw, the handle portion rotatably connected with a proximal end of the jaw portion; positioning a suture at the distal end of the lower jaw; actuating a jaw lever of the handle portion to cause the upper and lower jaws to close on a portion of tissue; and actuating a handle lever of the handle portion to pass a needle assembly along the axis through the lower jaw to pass a portion of the suture through the portion of tissue.

In accordance with certain aspects of this third aspect, the step of rotating may include engaging at least one spring-assisted bearing of the jaw portion with at least one depression of the handle portion in a first orientation of the instrument that temporarily locks the jaw portion with respect to handle portion. The method may further include the step of further rotating the handle portion with respect to the jaw portion from the orientation to a second orientation of the instrument that temporarily locks the jaw portion with respect to handle portion. The step of further rotating may include applying a force to overcome the connection between the at least one spring-assisted bearing and the at least one depression in the first temporarily locked orientation. The step of further rotating may occur after the step of actuating the jaw lever. The step of rotating may include rotating the handle portion with respect to the needle assembly about the axis.

The lower jaw may have a forked end, and the step of positioning may include positioning a suture between arms of the forked end for engagement with the needle assembly. The step of actuating the handle lever may include positioning the suture within a notch in the needle assembly. The method may further include the step of loading the needle assembly into the instrument by sliding a distal end of the needle assembly through the jaw portion and engaging a proximal end of the needle assembly with the handle portion. The method may further include the steps of releasing the handle lever to retract the needle assembly from the portion of tissue and releasing the jaw lever to release the grasp of the upper and lower jaws on the portion of tissue. The method may further include the step of positioning the upper and lower jaws adjacent to the portion of the suture passed through the tissue and actuating the jaw lever to cause the upper and lower jaws to close on the portion of the suture.

### BRIEF DESCRIPTION OF THE DRAWINGS

A more complete appreciation of the subject matter of the present invention and the various advantages thereof can be realized by reference to the following detailed description in which reference is made to the accompanying drawings in which:
FIG. 1 is a front perspective view of a suture passer assembly assembled with a needle assembly in accordance with an aspect of the present invention.
FIG. 2 is a front perspective view of the suture passer assembly shown in FIG. 1.
FIG. 3 is a side elevational view of the suture passer assembly shown in FIG. 1.
FIG. 4 is a side sectional view of the suture passer assembly shown in FIG. 1 taken along the line 4-4.
FIG. 5 is a side partial-sectional view of a handle portion of the suture passer assembly shown in FIG. 1.
FIG. 6 is a side sectional view of a shaft portion and a jaw portion of the suture passer assembly shown in FIG. 1.
FIG. 7 is a side sectional view of the proximal end of the shaft portion shown in FIG. 6.
FIG. 8 is a side sectional view of the jaw portion and the distal end of the shaft portion shown in FIG. 6.
FIG. 9 is a front perspective view of the jaw portion and the distal end of the shaft portion shown in FIG. 6.
FIG. 10 is a top perspective view of a bottom jaw with a needle guide of the suture passer assembly shown in FIG. 1.
FIG. 11 is a side sectional view of the connection between the handle portion and the shaft portion of the suture passer assembly shown in FIG. 1.
FIG. 12 is a top perspective view of a rotator and a lock nut of the suture passer assembly shown in FIG. 1.
FIG. 13 is a top perspective sectional view of the rotator and lock nut shown in FIG. 12 taken along the line 13-13.
FIG. 14 is a top perspective view of a collar of the suture passer assembly shown in FIG. 1.
FIG. 15 is a top view of an upper jaw of the suture passer assembly shown in FIG. 1.
FIG. 16 is a top perspective view of the upper jaw shown in FIG. 15.
FIG. 17 is a top perspective view of the needle assembly of the suture passer assembly shown in FIG. 1.
FIG. 18 is a top perspective view of a needle blade of the needle assembly shown in FIG. 17.
FIG. 19 is a top perspective view of a needle knob of the needle assembly shown in FIG. 17.
FIG. 20 is a top perspective view of the distal end of the needle blade shown in FIG. 18.

### DETAILED DESCRIPTION

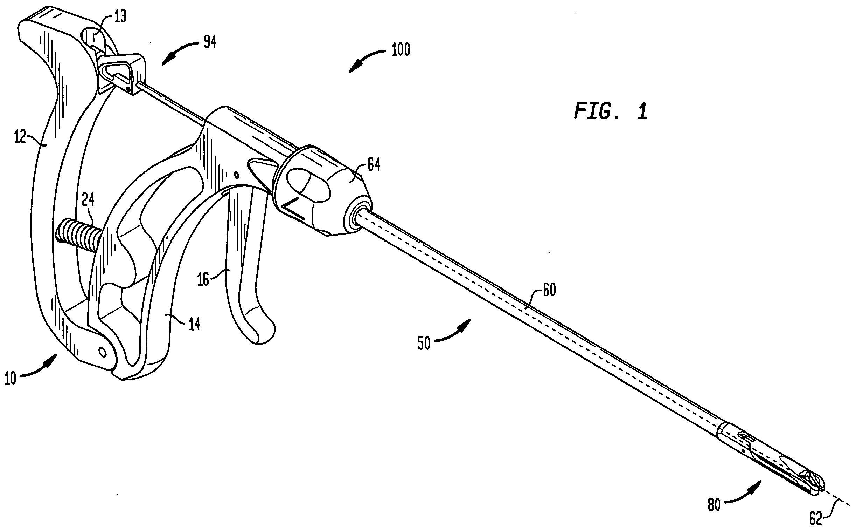

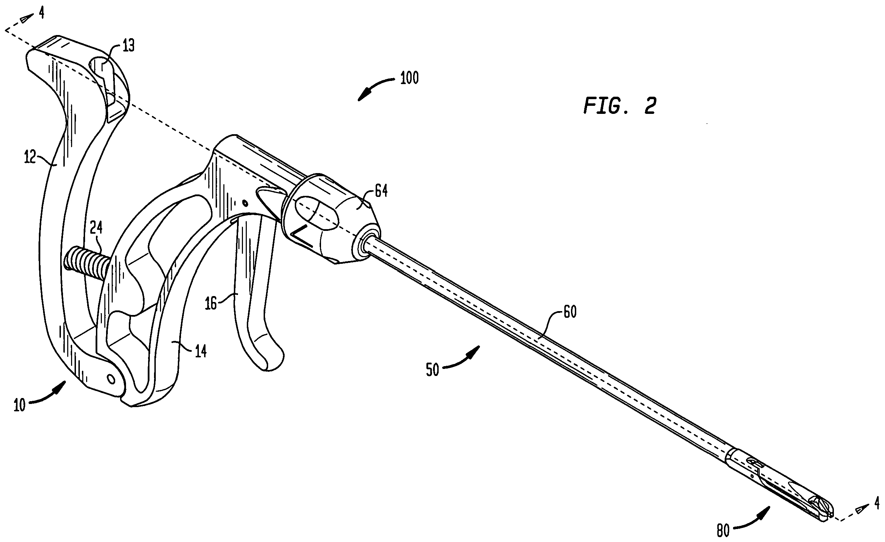

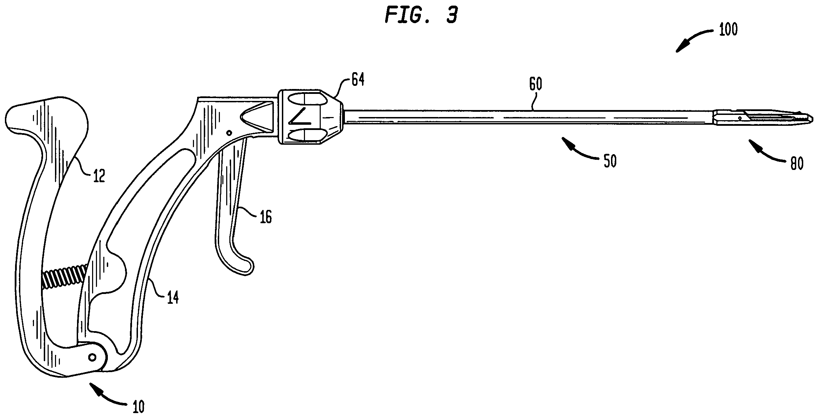

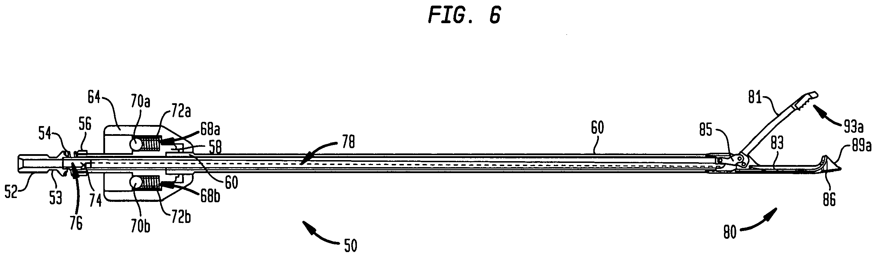

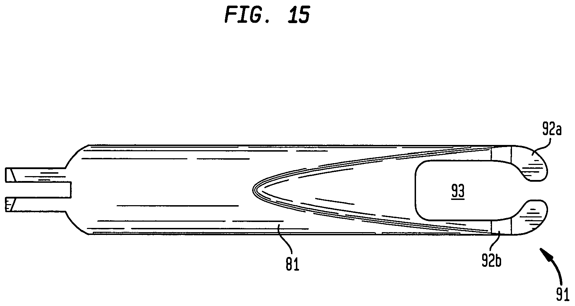

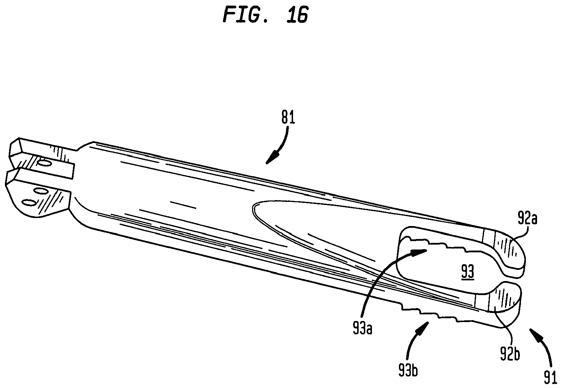

FIGS. 1-20 show a suture passer assembly 100 and components thereof, where assembly 100 includes a handle portion 10, a shaft portion 50, a jaw portion 80, and a needle assembly 94. Jaw portion 80 is depicted in a closed configuration in FIGS. 1-4 and in an open configuration in FIGS. 6, 8, and 9. Needle assembly 94 is adapted to be operated by handle portion 10, extend through shaft portion 50, and cooperate with jaw portion 80 to pass a suture through tissue. Shaft portion 50 is coupled to jaw portion 80, and as a unit, shaft portion 50 and jaw portion 80 are rotatably connected with handle portion 10. This allows shaft portion 50 and jaw portion 80 to be rotated with respect to handle portion 10, which rotation occurs about a central axis 62 (shown in FIG. 1) of a shaft 60 of shaft portion 50. Thus, a surgeon may ergonomically perform a suturing procedure by grasping and operating handle portion 10 from any number of locations about axis 62 that are independent of the position and orientation in which jaw portion 80 opens and closes. FIG. 2 shows suture passer assembly 100 without needle assembly 94.

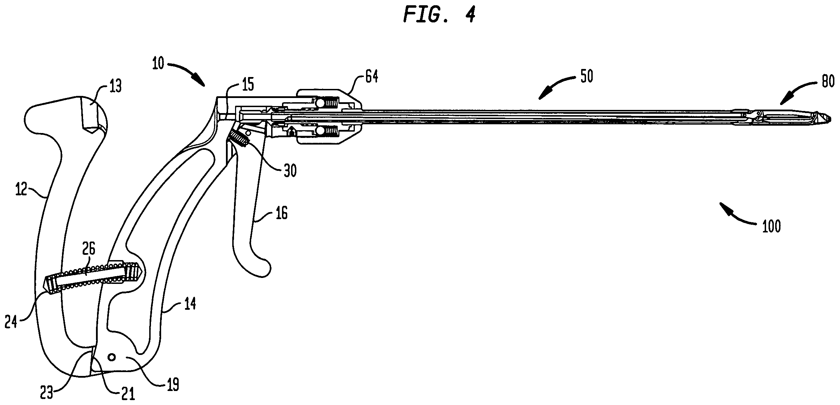

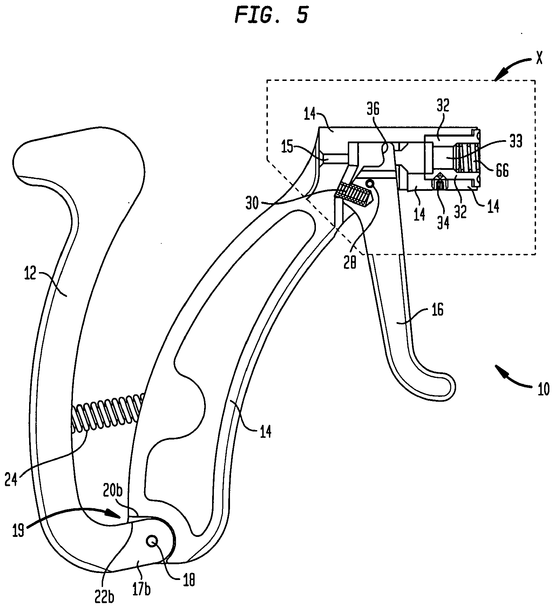

As shown in FIG. 5, which includes a sectional view within dashed region X, handle portion 10 includes handle lever 12, handle body 14, and jaw lever 16. Handle lever 12 is the proximal-most component of suture passer assembly 100 and is pivotally connected to handle body 14 through handle pin 18. As shown in FIGS. 4 and 5, handle lever 12 includes a first lever arm 17a (not shown) opposite a second lever arm 17b. Arms 17a, 17b are connected to either side of a lower portion 19 of handle body 14, such that, in order, handle pin 18 extends through lever arm 17a, lower portion 19, and lever arm 17b. A cavity 13, as shown in FIGS. 1, 2, and 4, is disposed in the upper portion of handle lever 12.

In its lower portion, handle body 14 includes first stops 20a (not shown) and 20b (shown in FIG. 5) adjacent lower portion 19. First stops 20a, 20b are configured to abut first walls 22a (not shown) and 22b of handle lever 12, which coincide respectively with the upper portions of lever arms 17a and 17b, when handle lever 12 and handle body 14 are pivoted toward one another. Likewise, as shown in FIG. 4, handle body 14 includes in the lower portion thereof a second stop 21 that is configured to abut a second wall 23 of handle lever 12 when handle lever 12 and handle body 14 are pivoted away from one another. Second wall 23 is positioned on handle lever 12 between lever arms 17a and 17b. First stops 20a, 20b and first walls 22a, 22b are generally horizontally oriented, while second stop 21 and second wall 23 are generally vertically oriented. Further, in its upper portion, handle body 14 includes a horizontal entry 15 generally aligned with axis 62 of shaft 60 in the assembled condition of assembly 100.

Disposed in a substantially horizontal orientation between handle lever 12 and handle body 14 are a handle spring 24 and a dowel pin 26, as shown in FIGS. 4 and 5. Handle spring 24 is normally biased to separate or force apart handle lever 12 and handle body 14 with respect to the axis of rotation at handle pin 18 into a position where second stop 21 contacts second wall 23. Dowel pin 26 is disposed within handle spring 24 to prevent handle spring 24 from bending or rotating in a direction perpendicular to the general direction of its axial compression/extension.

Jaw lever 16 is connected to handle body 14 via jaw lever pin 28, and is similarly biased to separate from handle body 14 with respect to the axis of rotation at lever pin 28 due to the force of a jaw lever spring 30. The stiffness in jaw lever spring 30 is preferably less than the stiffness in handle spring 24. The result of the differing stiffnesses causes jaw lever 16 to move relative to handle body 14 before handle lever 12 moves relative to handle body 14 when the three components are squeezed together by a surgeon's hand, that is, when forces are applied to handle lever 12 and jaw lever 16 simultaneously in an effort to move them toward one another. Conversely, the result of the differing stiffnesses causes handle lever 12 to move relative to handle body 14 before jaw lever 16 moves relative to handle body 14 when the three components are released by the surgeon's hand. Essentially, when a surgeon squeezes and subsequently releases handle portion 10, the operation of assembly 100 will be: (1) jaw portion 80 closes; (2) needle assembly 94 moves distally toward jaw portion 80 to engage the tissue; (3) needle assembly 94 retracts proximally from jaw portion 80; and (4) jaw portion 80 opens. Thus, the construction of suture passer assembly 100 ensures that jaw portion 80 will be closed during needle engagement and retraction, i.e., movement of needle assembly 94.

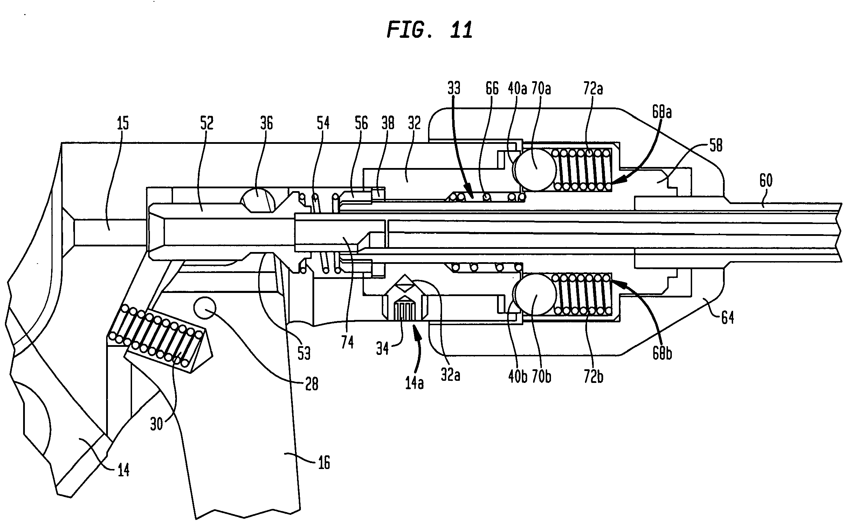

As shown in FIGS. 4, 5, 11, and 14, a collar 32 is disposed within and rotationally coupled to the distal-most portion of handle body 14. A set screw 34 is screwed through a screw bore 14a in handle body 14 and into a set screw bore 32a in collar 32, which is shown more clearly in FIG. 11. Each of the above-described elements of handle portion 10 are configured such that handle portion 10 rotates about axis 62 of shaft 60 as a single unit.

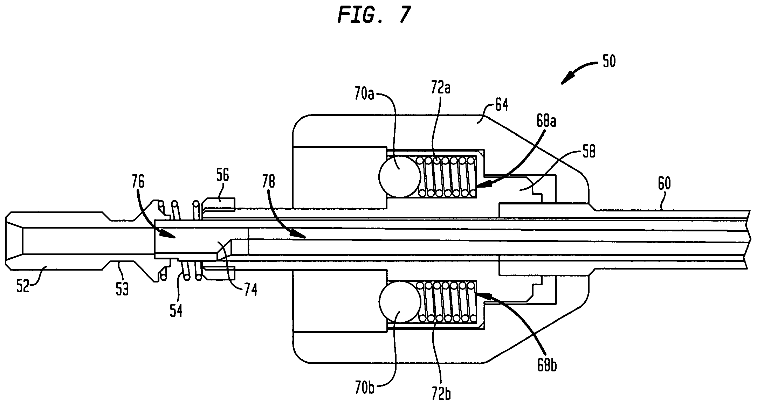

As shown in FIGS. 6 and 7, shaft portion 50 includes a jaw actuator connector 52, a jaw spring 54, a lock nut 56, a jaw actuator 74, and shaft 60. Jaw actuator connector 52 includes a necked down area at a neck 53 that engages with a forked end 36 of jaw lever 16, as shown in FIG. 11. Jaw actuator connector 52 abuts and is connected to the proximal end of jaw actuator 74, which extends through shaft 60. Jaw spring 54 is disposed about the proximal end of jaw actuator 74 and between the distal end of jaw actuator connector 52 and lock nut 56. As shown in FIG. 11, lock nut 56 presses against washer 38, which in turn presses against the proximal portion of collar 32. When a distal force is applied to jaw actuator connector 52, lock nut 56 is prevented from moving distally relative to handle portion 10 when it is pressed against collar 32 via washer 38, and jaw spring 54 is therefore compressed. Such a distal force also causes jaw actuator 74 to move distally within shaft 60.

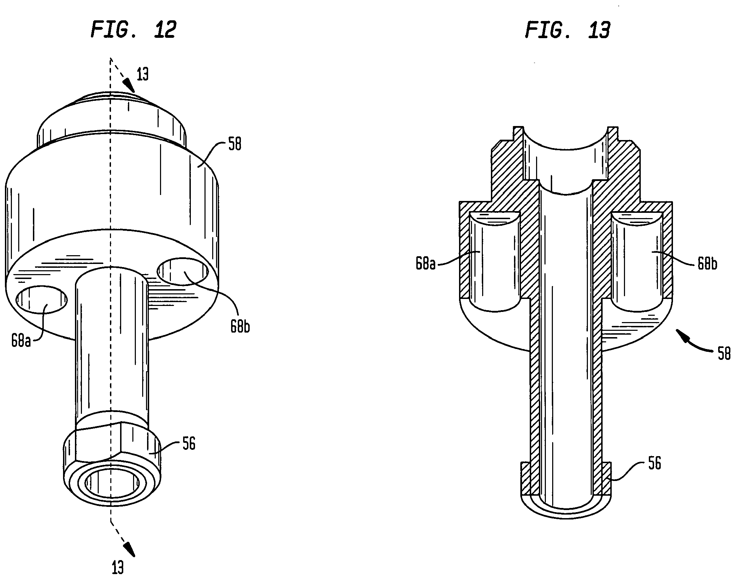

Shaft portion 50 further includes a rotator 58 and a rotator knob 64, as shown in FIGS. 6, 7, and 11. A portion of rotator 58 is disposed within a central bore 33 of collar 32, and jaw actuator 74 extends through rotator 58. The proximal end of rotator 58 is fitted with lock nut 56 (also shown in FIGS. 12 and 13), which contacts the proximal portion of collar 32 via washer 38 and prevents rotator 58 from moving distally with respect to collar 32 when lock nut 56 contacts washer 38. A collar spring 66 is disposed within the distal portion of collar 32 and is biased to extend between adjacent internal faces of collar 32 and rotator 58 to force such elements apart from one another. Again, lock nut 56 and washer 38 prevent separation between collar 32 and rotator 58. However, axial rotation between collar 32 and rotator 58 is permitted.

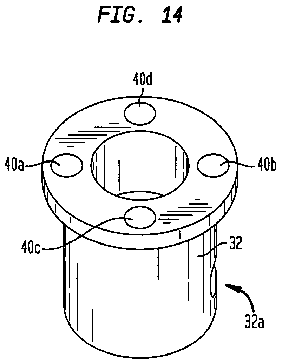

The distal, wider portion of rotator 58 includes two proximally facing bores 68a, 68b, as shown in FIGS. 12 and 13, which each may house bearings or balls 70a, 70b and ball springs 72a, 72b. Within bores 68a, 68b, balls 70a, 70b are located proximally of ball springs 72a, 72b, which act against an inside face of bores 68a, 68b to force balls 70a, 70b in a proximal direction. On the distal face of collar 32 are depressions or detents 40a, 40b, as shown in FIGS. 11 and 14, which are indentations in collar 32 that mate with a surface of balls 70a, 70b. When balls 70a, 70b are positioned adjacent to detents 40a, 40b, ball springs 72a, 72b act to force balls 70a, 70b into detents 40a, 40b, thus creating a spring-assisted ball or bearing. The force by which balls 70a, 70b are held within detents 40a, 40b by ball springs 72a, 72b act to temporarily lock and substantially prevent rotation of rotator 58 with respect to collar 32, and accordingly shaft portion 50 with respect to handle portion 10. Substantial prevention of the rotation of shaft portion 50 with respect to handle portion 10 does not require complete prevention of rotation, but rather requires enough connection between the portions to prevent rotation due to the normal use of the device by the surgeon during a surgical procedure, thus creating a temporary locked position of assembly 100. Handle portion 10 can be further rotated with respect to shaft portion 50 and jaw portion 80 through application of a force to overcome the connection between the spring-assisted balls 70a, 70b and depressions 40a, 40b in the temporarily locked orientation.

The present invention preferably includes four detents 40a, 40b, 40c, 40d to allow for positioning of rotator 58 into four particular positions with respect to collar 32. However, there may be any number of detents 40 in collar 32. The present invention also allows for any number of bores 68 and corresponding balls 70 and ball springs 72 in rotator 58. The number of balls 70 may be greater than, equal to, or less than the number of detents 40 in suture passer assembly 100. Further, the stiffness of ball spring 72 may be chosen according to the amount of force desired to hold collar 32 with respect to rotator 58. The ease of rotation between handle portion 10 and shaft portion 50 may therefore be determined by the components. While the depicted embodiment of assembly 100 includes four "locked" positions each 90 degrees from one another about axis 62, it will be understood that shaft portion 50 can be rotated and "locked" with respect to handle portion 10 in any desirable number of positions, such as every 15 degrees, 30 degrees, 45 degrees, 60 degrees, 180 degrees, etc.

As shown in FIGS. 6 and 7, jaw actuator 74 includes a proximal cylindrical portion 76 and a distal U-shaped portion 78. Proximal cylindrical portion 76 is disposed against jaw actuator connector 52 and is relatively shorter than distal U-shaped portion 78, which extends toward jaw portion 80.

The proximal end of shaft 60 is rotationally coupled within the distal end of rotator 58, as shown in FIG. 7, and may be connected thereto through a weld. Rotator knob 64 is also rotationally coupled to shaft 60 and may be welded thereto. An adjacent connection between rotator knob 64 and rotator 58 may also be welded.

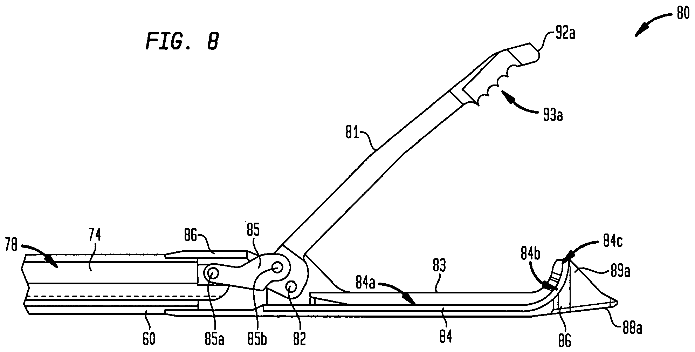

As shown in FIGS. 8-10, jaw portion 80 includes an upper jaw 81 and a lower jaw 86, which is cylindrical at its proximal end and welded thereat about shaft 60. Upper jaw 81 is pivotally connected to lower jaw 86 via a jaw pin 82, which is located distally of shaft 60. A needle guide 83 is connected to lower jaw 86, preferably by welding, to provide a needle track 84, which is curved upward at its distal end.

Upper jaw 81 is connected to jaw actuator 74 through a jaw link 85, shown in FIG. 8. Jaw link 85 is pivotally connected to jaw actuator 74 through a first pin 85a and is pivotally connected to upper jaw 81 through a second pin 85b. Thus, jaw link 85 allows for a translational movement of jaw actuator 74 to be converted into pivotal movement of upper jaw 81, such pivotal movement being about jaw pin 82. Movement of jaw actuator 74 therefore allows upper jaw 81 to open and close with respect to lower jaw 86.

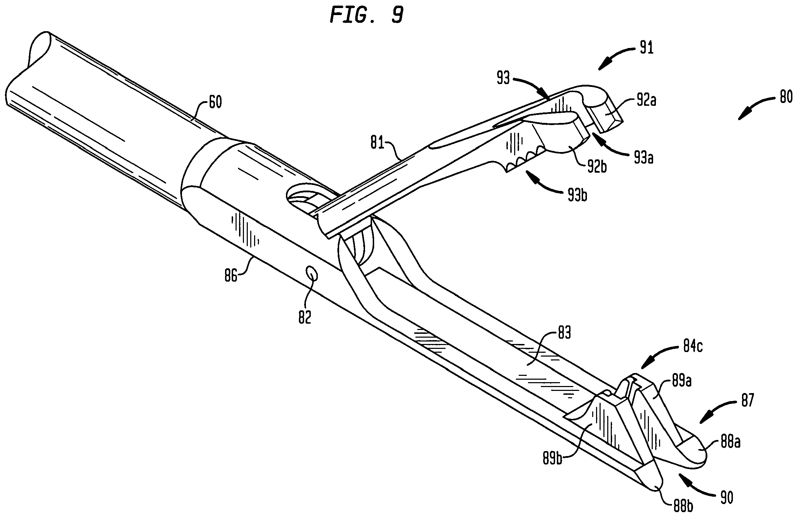

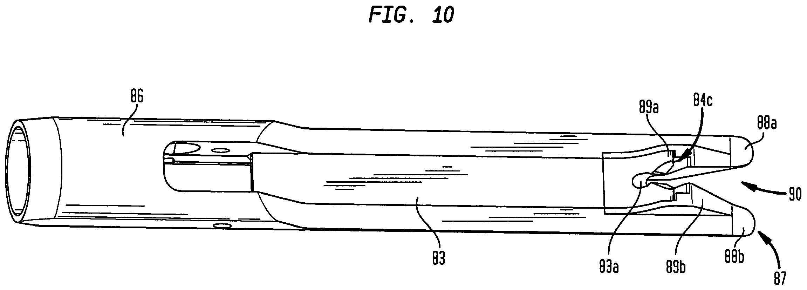

Lower jaw 86 includes a forked end 87 split into a first arm 88a and a second arm 88b, as shown in FIGS. 9 and 10. Each arm 88a, 88b includes a rising portion 89a, 89b through which the lateral edges of needle track 84 extend. Needle track 84 thus includes a horizontal portion 84a, a curved portion 84b, and a vertical exit portion 84c. Defined by forked end 87, a space 90 extends proximally into lower jaw 86 between rising portions 89a and 89b. Space 90 extends proximally of vertical exit portion 84c and at least partially through curved portion 84b, as shown in FIG. 10. Needle guide 83 also includes a guide notch 83a that extends into needle guide 83 along vertical exit portion 84c and at least a portion of curved portion 84b. Guide notch 83a communicates with space 90.

As shown in FIGS. 9, 15, and 16, upper jaw 81 includes a forked end 91 having a first arm 92a and a second arm 92b. The distalmost portions of arms 92a and 92b extend towards one another to define a space 93 between arms 92a and 92b. The bottom face of each of arms 92a and 92b includes a set of teeth 93a, 93b which aid in gripping tissue disposed between upper jaw 81 and lower jaw 86. Further, arms 92a and 92b of upper jaw 81 are disposed to be seated outside, or to at least partially overlap in a horizontal plane, the respective rising portions 89a and 89b when upper jaw 81 is closed with respect to lower jaw 86.

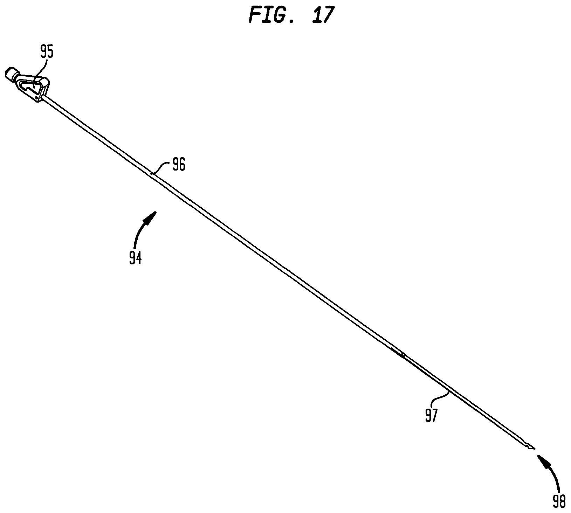

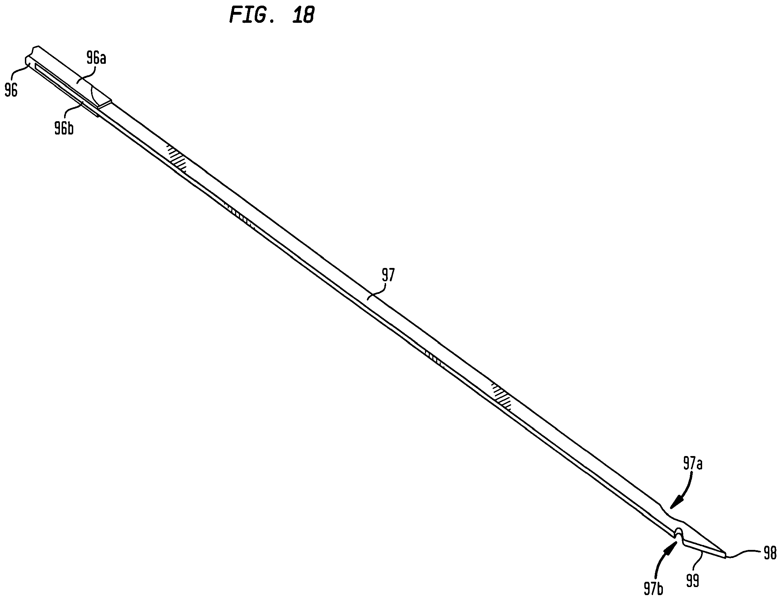

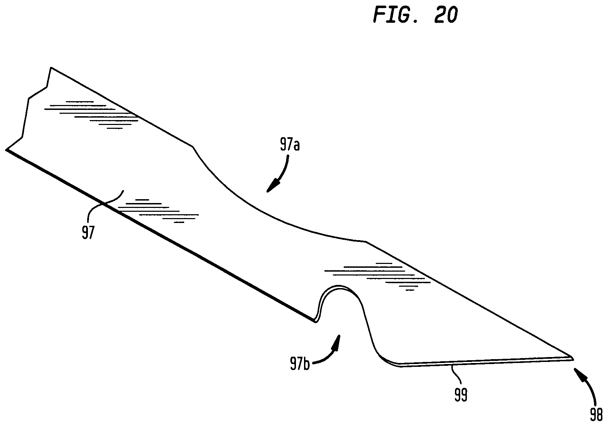

Shown in FIGS. 17-20, needle assembly 94 includes a needle grip 95, a needle shaft 96, and a needle blade 97. At least needle blade 97 is preferably flexible, yet rigid enough to pierce tissue without breaking. Needle blade 97 is configured in a flat, non-circular construction that cooperates with the relatively flat and wide, non-circular configuration of needle track 84. As shown in FIGS. 18 and 20, the distal portion of needle blade 97 includes a relief 97a, a suture notch 97b, and a tip 98. Suture notch 97b is configured to engage a suture initially disposed in space 90 of lower jaw 86 after needle blade 97 and suture notch 97b pass through and exit needle track 84.

As shown in FIG. 20, relief 97a is provided so that the distal portion of needle blade 97 may allow for bending in a medial-lateral direction without breaking due to stress or fatigue in needle blade 97. Suture notch 97b is generally configured to extend in from the side of needle blade 97 in a medial/proximal direction so as to carry a suture disposed within suture notch 97b in a distal direction. Needle blade 97 further has an edge 99 that extends proximally from tip 98 to the alternate side of needle blade 97, and towards the side of needle blade 97 that includes suture notch 97b. In this configuration, edge 99 is disposed distally of suture notch 97b such that when needle blade 97 is passed through tissue, edge 99 cuts a hole or slot in the tissue so that any proximally-positioned portions of needle blade 97 easily pass through the tissue.

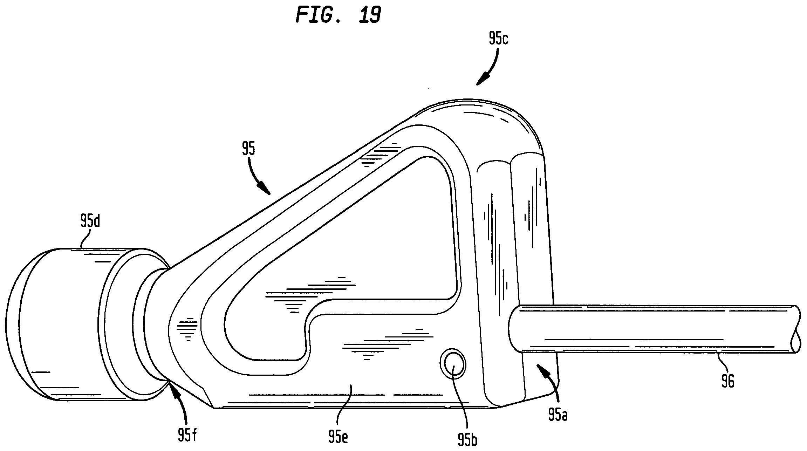

The proximal end of needle blade 97 is disposed between two legs 96a and 96b of needle shaft 96, as shown in FIG. 18. The proximal end of needle shaft 96 is disposed within a bore 95a of needle grip 95, as shown in FIG. 19, and may be secured within bore 95a via cross pin 95b. Needle grip 95 includes a body 95e having a protrusion 95c, a neck 95f, and a knob 95d. Each component of needle assembly 94 is rotationally coupled, and protrusion 95c therefore gives an indication as to the direction that the superior surface of needle blade 97 faces. Knob 95d fits within cavity 13 of handle lever 12, which allows needle assembly 94 to move in a proximal-distal direction with respect to jaw portion 80 according to the position of handle lever 12. Knob 95d and neck 95f are adapted to allow for the rotation of needle assembly 94 relative to handle portion 10. Neck 95f of needle grip 95 fits into the edge of cavity 13 in handle lever 12 and allows the needle to rotate about an axis coincident to or generally parallel to axis 62 while still being able to be actuated in the proximal-distal direction by handle lever 12. The interference fit of knob 95d and neck 95f with cavity 13 allows for proximal and distal forces to be applied to needle assembly 94, while the general axial nature of needle assembly 94 allows for rotation of same with respect to handle portion 10. The components of needle assembly 94 may be flexible to allow for the above-described movement.

In use, a surgeon may load needle assembly 94 into handle portion 10, shaft portion 50, and jaw portion 80, or needle assembly 94 may be packaged in a pre-assembled manner. Needle assembly 94 is loaded by threading tip 98 through entry 15 of handle body 14, and sliding needle assembly 94 distally through shaft 60 and along axis 62. Once needle grip 95 is disposed above cavity 13 in handle lever 12, needle grip 95 is lowered into cavity 13 and is retained due to the relatively rigid nature needle assembly 94 disposed within shaft portion 50, which maintains needle grip 95 in its position within cavity 13. Needle grip 95 may be additionally removeably secured within cavity 13 by a press fit interference. Needle assembly 94 is thus loaded and ready to be passed through tissue.

The surgeon may then select the rotational orientation of handle portion 10 with respect to shaft portion 50 and jaw portion 80. This is accomplished by grasping rotator knob 64 and handle portion 10, and rotating one with respect to the other. When the surgeon rotates rotator knob 64 to overcome the force of ball springs 72a, 72b, balls 70a, 70b disengage from adjacent detents 40a, 40b and may be rotated into position adjacent to alternate detents 40. Of course, it is possible for suture passer assembly 100 to be utilized without a mating connection between balls 70 and detents 40. The surgeon will receive tactile feedback when a mating engagement or release between balls 70 and detents 40 occurs. It will be understood that the configuration of additional balls 70 and detents 40 may allow suture passer assembly 100 to have additional "lockable" positions. The flat construction of needle blade 97 and the similarly flat construction of the cross section of needle track 84 allows for rotational coupling between jaw portion 80 and needle assembly 94. Thus, when the coupled shaft portion 50 and jaw portion 80 are rotated with respect to handle portion 10, such rotation also rotates needle assembly 94 so that it maintains its alignment and orientation with respect to shaft portion 50 and jaw portion 80, thereby causing knob 95d to rotate within cavity.

Of course, the rotatable nature of suture passer assembly 100 allows for rotation to occur between the respective portions either before, during, or after a procedure. For reasons that will be understood by those of ordinary skill in the art, the positioning of jaw portion 80 during a surgical procedure may make it difficult to operate suture passer assembly 100 if handle portion 10 is positioned in a manner that makes suture passer assembly 100 difficult to grasp for the surgeon. If a more suitable rotational position of handle portion 10 is anticipated prior to surgery, the surgeon can place handle portion 10 in such position at that time. However, if it becomes clear during surgery that a different position of handle portion 10 is more advantageous, such rotation can occur while the procedure is in progress.

With needle assembly 94 preferably in its operative position with respect to handle portion 10, a suture may be positioned within space 90 of lower jaw 86, and in particular, within guide notch 83a. The suture is preferably disposed such that one end hangs through space 90 and below lower jaw 86. The other end of the suture is positioned to one side of lower jaw 86 proximally of either of rising portions 89a or 89b. Therefore, the suture is positioned to intersect needle track 84 at some point along curved portion 84b. The suture is thus in position to be picked up by needle blade 97, and more specifically, within suture notch 97b, when needle assembly 94 is passed distally with respect to jaw portion 80. A suture may alternatively be disposed proximate to the surgical site, and lower jaw 86 can be navigated during the procedure to position the suture within space 90 of lower jaw 86 prior to deployment of needle assembly 94.

With the suture in position with respect to suture passer assembly 100, the surgeon navigates jaw portion 80 toward the tissue to be sutured. Typically, this occurs via a cannula to the surgical site. While jaw portion 80 is biased to be in an open configuration when no pressure is applied by the surgeon to jaw lever 16, the surgeon preferably pulls jaw lever 16 to close jaw portion 80 to reduce its profile when jaw portion 80 is navigated toward the surgical site. This also helps to more securely hold the suture in its position within space 90 of lower jaw 86. Once at the surgical site, jaw portion 80 can be opened by the surgeon releasing pressure on jaw lever 16. After upper jaw 81 and lower jaw 86 are appropriately positioned with respect to the tissue, jaw lever 16 is pulled proximally by the surgeon, thereby closing upper jaw 81 with respect to lower jaw 86. This is accomplished as jaw lever 16 pivots about jaw lever pin 28 and forked end 36 of jaw lever 16 forces jaw actuator connector 52 in the distal direction, which in turn moves jaw actuator 74, jaw link 85, and upper jaw 81.

When upper jaw 81 closes with respect to lower jaw 86, the tissue is clamped therebetween, and teeth 93a, 93b aid in grasping tissue. Space 93 of upper jaw 81 is disposed immediately above rising portions 89a, 89b of lower jaw 86 such that at least a portion of the tissue is forced into space 93, further enhancing the exact grip on the tissue and applying tension to the area of tissue to be punctured by needle blade 97. In this configuration, vertical exit portion 84c of needle track 84 is preferably positioned adjacent to the intended puncture site in the tissue.

While the surgeon maintains the position of the tissue by maintaining his grasp of jaw lever 16, the surgeon further squeezes handle portion 10, pivoting handle lever 12 with respect to handle body 14 and thereby advancing needle assembly 94 distally. Needle blade 97 is advanced along horizontal portion 84a of needle track 84, through curved portion 84b and through vertical exit portion 84c where tip 98 pierces the tissue. Once suture notch 97b passes through and exits vertical exit portion 84c, it encounters the suture disposed in space 90 and the suture enters suture notch 97b. Further advancement of needle assembly 94 passes suture notch 97b and the suture through the punctured hole in the tissue.

At this point with the suture disposed through the tissue, the surgeon relaxes his grip on handle portion, which allows handle spring 24 to force handle lever 12 in a distal direction, which thereby retracts needle assembly 94 from the tissue. However, the suture remains disposed through the punctured hole in the tissue as needle blade 97 is retracted through the punctured hole and back into needle track 84. The surgeon then further relaxes his grip on handle portion 10, allowing jaw lever spring 30 to force jaw lever 16 in a proximal direction, which thereby opens upper jaw 81 with respect to lower jaw 86 and releases the grasp of jaw portion 80 on the tissue. Suture passer assembly 100 is then pulled proximally and away from the tissue, while the suture is left positioned through the puncture hole in the tissue.

After suture passer assembly 100 has been retracted, the passed portion of the suture can be captured via jaw portion 80 of suture passer assembly 100. Otherwise, an additional tool can be utilized by the surgeon to capture the passed suture, either during or after operation of suture passer assembly 100.

Although the invention herein has been described with reference to particular aspects it is to be understood that these embodiments are merely illustrative of the principles and applications of the present invention. It is therefore to be understood that numerous modifications may be made and are encouraged to be made to the illustrative aspects and that other arrangements may be devised without departing from the scope of the present invention as defined by herein disclosure and/or the appended claims.

## Claims

1. A suture passer assembly (100) comprising:
a jaw portion (80) extending along an axis (62) and having a proximal end;
a handle portion (10) rotatably connected with the proximal end of the jaw portion (80) such that the handle portion (10) is configured to fully rotate about the axis (62) with respect to the jaw portion (80), and
a needle assembly (94) configured to pass through the jaw portion (80) and adapted to be passed along the axis (62) by cooperation of the needle assembly (94) with the handle portion (10), said suture passer assembly being **characterized in that** the handle portion (10) includes a handle lever (12), **in that** a cavity (13) is disposed in an upper portion of the handle lever (12), **in that** the needle assembly (94) has a needle grip (95) including a neck (95f) and a knob (95b) and **in that** the knob (95d) fits within the cavity (13) of the handle lever (12), which allows the needle assembly (94) to move along the axis (62) according to the position of the handle lever (12), and
the neck (95f) of the needle grip (95) fits into the edge of the cavity (13) in the handle lever (12) and allows the needle assembly (94) to rotate about an axis coincident to or generally parallel to the axis (62).

2. The assembly (100) of claim 1, further comprising a shaft portion (50) extending along the axis (62) between the handle portion (10) and the jaw portion (80), the shaft portion (50) being rotationally coupled to the proximal end of the jaw portion (80).

3. The assembly (100) of claim 1, wherein the jaw portion (80) has a distal end, the distal end having an upper jaw (81) and a lower jaw (86) having a forked end (87).

4. The assembly (100) of claim 3, further comprising a shaft portion (50) extending along the axis (62) between the handle portion (10) and the jaw portion (80), the shaft portion (50) being rotationally coupled to the proximal end of the jaw portion (80), and wherein the needle assembly (94) is configured to pass through the shaft portion (50) and the lower jaw (86).

5. The assembly (100) of claim 3, wherein the lower jaw (86) includes a non-circular needle channel, and the needle assembly (94) includes a non-circular needle blade slidable within the needle channel, wherein the needle assembly (94) is rotationally coupled with the jaw portion (80) and rotationally connected to the handle portion (10) due to the mating configuration between the needle channel and needle blade.

6. The assembly (100) of claim 3, wherein the lower jaw (86) includes a needle channel and the forked end (36) defines first and second arms (17) having a space therebetween that intersects the needle channel, wherein the space is configured to accept a suture for engagement with the needle assembly (94).

7. The assembly (100) of claim 2, wherein the shaft portion (50) includes at least one spring-assisted bearing (70) and the handle portion (10) includes at least one depression (40) configured to engage the at least one spring-assisted bearing (70) in an orientation that temporarily locks the shaft portion (50) with respect to the handle portion (10).

8. The assembly (100) of claim 7, wherein the at least one spring-assisted bearing (70) and the at least one depression (40) are engageable in a finite number of orientations about the axis that temporarily lock the shaft portion (50) with respect to the handle portion (10).

9. The assembly (100) of claim 7, wherein the handle portion (10) can be further rotated with respect to the jaw portion (80) through application of a force to overcome the connection between the at least one spring-assisted bearing (70) and the at least one depression (40) in the temporarily locked orientation.

## Patentansprüche

1. Nahtfadendurchführungsanordnung (100), die Folgendes umfasst:
einen Backenteil (80), der sich entlang einer Achse (62) erstreckt und ein proximales Ende aufweist;
einen Griffteil (10), der drehbar mit dem proximalen Ende des Backenteils (80) verbunden ist, so dass der Griffteil (10) dazu konfiguriert ist, sich vollständig um die Achse (62) in Bezug auf den Backenteil (80) zu drehen, und
eine Nadelanordnung (94), die dazu konfiguriert ist, durch den Backenteil (80) durchzutreten, und dazu eingerichtet ist, entlang der Achse (62) durch Zusammenwirkung der Nadelanordnung (94) mit dem Griffteil (10) geführt zu werden,
wobei die Nahtfadendurchführungsanordnung **dadurch gekennzeichnet ist, dass** der Griffteil (10) einen Griffhebel (12) beinhaltet, dass eine Aussparung (13) in einem oberen Teil des Griffhebels (12) angeordnet ist, dass die Nadelanordnung (94) einen Nadelhaltegriff (95) aufweist, der einen Ansatz (95f) und einen Knauf (95b) beinhaltet, und dass der Knauf (95d) in die Aussparung (13) des Griffhebels (12) passt, was der Nadelanordnung (94) ermöglicht, sich entlang der Achse (62) entsprechend der Position des Griffhebels (12) zu bewegen, und
der Ansatz (95f) des Nadelhaltegriffs (95) in den Rand der Aussparung (13) in dem Griffhebel (12) passt und der Nadelanordnung (94) ermöglicht, sich um eine Achse zu drehen, die mit der Achse (62) zusammenfällt oder im Allgemeinen parallel zu dieser ist.

2. Anordnung (100) nach Anspruch 1, die weiterhin einen Schaftteil (50) umfasst, der sich entlang der Achse (62) zwischen dem Griffteil (10) und dem Backenteil (80) erstreckt, wobei der Schaftteil (50) mit dem proximalen Ende des Backenteils (80) drehgekoppelt ist.

3. Anordnung (100) nach Anspruch 1, wobei der Backenteil (80) ein distales Ende aufweist, wobei das distale Ende eine obere Backe (81) und eine untere Backe (86) mit einem gespaltenen Ende (87) aufweist.

4. Anordnung (100) nach Anspruch 3, die weiterhin einen Schaftteil (50) umfasst, der sich entlang der Achse (62) zwischen dem Griffteil (10) und dem Backenteil (80) erstreckt, wobei der Schaftteil (50) mit dem proximalen Ende des Backenteils (80) drehgekoppelt ist und wobei die Nadelanordnung (94) dazu konfiguriert ist, durch den Schaftteil (50) und die untere Backe (86) durchzutreten.

5. Anordnung (100) nach Anspruch 3, wobei die untere Backe (86) einen nicht kreisförmigen Nadelkanal beinhaltet und die Nadelanordnung (94) einen nicht kreisförmigen Nadelschaft beinhaltet, der innerhalb des Nadelkanals verschiebbar ist, wobei die Nadelanordnung (94) mit dem Backenteil (80) drehgekoppelt ist und aufgrund der Passungskonfiguration zwischen dem Nadelkanal und dem Nadelschaft mit dem Griffteil (10) drehverbunden ist.

6. Anordnung (100) nach Anspruch 3, wobei die untere Backe (86) einen Nadelkanal beinhaltet und das gespaltene Ende (36) einen ersten und einen zweiten Arm (17) definiert, die einen Raum dazwischen aufweisen, der den Nadelkanal durchschneidet, wobei der Raum dazu konfiguriert ist, einen Nahtfaden zum Eingriff mit der Nadelanordnung (94) aufzunehmen.

7. Anordnung (100) nach Anspruch 2, wobei der Schaftteil (50) mindestens ein federgestütztes Lager (70) beinhaltet und der Griffteil (10) mindestens eine Vertiefung (40) beinhaltet, die dazu konfiguriert ist, das mindestens eine federgestützte Lager (70) in einer Ausrichtung in Eingriff zu nehmen, die den Schaftteil (50) in Bezug auf den Griffteil (10) vorübergehend arretiert.

8. Anordnung (100) nach Anspruch 7, wobei das mindestens eine federgestützte Lager (70) und die mindestens eine Vertiefung (40) in einer endlichen Anzahl von Ausrichtungen um die Achse in Eingriff gebracht werden können, die den Schaftteil (50) in Bezug auf den Griffteil (10) vorübergehend arretieren.

9. Anordnung (100) nach Anspruch 7, wobei der Griffteil (10) weiterhin in Bezug auf den Backenteil (80) durch Ausübung einer Kraft gedreht werden kann, um die Verbindung zwischen dem mindestens einen federgestützten Lager (70) und der mindestens einen Vertiefung (40) in der vorübergehend arretierten Ausrichtung zu überwinden.

## Revendications

1. Ensemble de passe-suture (100) comprenant :
une portion de mâchoire (80) s'étendant le long d'un axe (62) et ayant une extrémité proximale ;
une portion de manche (10) raccordée en rotation à l'extrémité proximale de la portion de mâchoire (80) de sorte que la portion de manche (10) soit configurée pour tourner totalement autour de l'axe (62) par rapport à la portion de mâchoire (80) ; et
un ensemble aiguille (94) configurés pour passer à travers la portion de mâchoire (80) et adapté pour être passé le long de l'axe (62) par coopération de l'ensemble aiguille (94) avec la portion de manche (10),
ledit ensemble passe-suture étant **caractérisé en ce que** la portion de manche (10) comporte un levier de manche (12), **en ce qu'**une cavité (13) est disposée dans une portion supérieure du levier de manche (12), **en ce que** l'ensemble aiguille (94) a un pince-aiguille (95) comportant un collet (95f) et un bouton (95b) et **en ce que** le bouton (95d) s'ajuste au sein de la cavité (13) du levier de manche (12), ce qui permet à l'ensemble aiguille (94) de se déplacer le long de l'axe (62) selon la position du levier de manche (12), et
le collet (95f) du pince-aiguille (95) s'ajuste dans le bord de la cavité (13) dans le levier de manche (12) et permet à l'ensemble aiguille (94) de tourner autour d'un axe coïncidant ou généralement parallèle à l'axe (62).

2. Ensemble (100) selon la revendication 1, comprenant en outre une portion de tige (50) s'étendant le long de l'axe (62) entre la portion de manche (10) et la portion de mâchoire (80), la portion de tige (50) étant couplée en rotation à l'extrémité proximale de la portion de mâchoire (80).

3. Ensemble (100) selon la revendication 1, dans lequel la portion de mâchoire (80) a une extrémité distale, l'extrémité distale ayant une mâchoire supérieure (81) et une mâchoire inférieure (86) ayant une extrémité fourchue (87).

4. Ensemble (100) selon la revendication 3, comprenant en outre une portion de tige (50) s'étendant le long de l'axe (62) entre la portion de manche (10) et la portion de mâchoire (80), la portion de tige (50) étant couplée en rotation à l'extrémité proximale de la portion de mâchoire (80), et dans lequel l'ensemble aiguille (94) est configuré pour passer à travers la portion de tige (50) et la mâchoire inférieure (86).

5. Ensemble (100) selon la revendication 3, dans lequel la mâchoire inférieure (86) comporte un canal à aiguille non circulaire, et l'ensemble aiguille (94) comporte une lame d'aiguille non circulaire coulissant au sein du canal à aiguille, dans lequel l'ensemble aiguille (94) est couplé en rotation à la portion de mâchoire (80) et raccordé en rotation à la portion de manche (10) en raison de la configuration d'appariement entre le canal à aiguille et la lame d'aiguille.

6. Ensemble (100) selon la revendication 3, dans lequel la mâchoire inférieure (86) comporte un canal à aiguille et l'extrémité fourchue (36) définit des premier et second bras (17) ayant un espace entre eux qui coupe le canal à aiguille, dans lequel l'espace est configuré pour accepter une suture pour s'engager avec l'ensemble aiguille (94).

7. Ensemble (100) selon la revendication 2, dans lequel la portion de tige (50) comporte au moins une butée à ressort (70) et la portion de manche (10) comporte au moins un creux (40) configuré pour s'engager avec l'au moins une butée à ressort (70) dans une orientation qui bloque temporairement la portion de tige (50) par rapport à la portion de manche (10).

8. Ensemble (100) selon la revendication 7, dans lequel l'au moins une butée à ressort (70) et l'au moins un creux (40) sont engageables dans un nombre fini d'orientations autour de l'axe qui bloquent temporairement la portion de tige (50) par rapport à la portion de manche (10).

9. Ensemble (100) selon la revendication 7, dans lequel la portion de manche (10) peut en outre être tournée par rapport à la portion de mâchoire (80) par l'application d'une force pour surmonter le raccordement entre l'au moins une butée à ressort (70) et l'au moins un creux (40) dans l'orientation temporairement bloquée.
